(19) Europäisches Patentamt
European Patent Office
Office européen des brevets

(11) **EP 4 372 355 A1**

(12) **EUROPEAN PATENT APPLICATION**
published in accordance with Art. 153(4) EPC

(43) Date of publication:
**22.05.2024  Bulletin 2024/21**

(21) Application number: **21949977.9**

(22) Date of filing: **16.11.2021**

(51) International Patent Classification (IPC):
***G01N 1/14*** *(2006.01)*

(52) Cooperative Patent Classification (CPC):
**G01N 1/14**

(86) International application number:
**PCT/CN2021/130879**

(87) International publication number:
**WO 2023/284205 (19.01.2023 Gazette 2023/03)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **16.07.2021  CN 202110809061**

(71) Applicant: **Jiangsu Leuven Instruments Co. Ltd
Xuzhou, Jiangsu 221300 (CN)**

(72) Inventors:
• **ZHANG, Huaidong
Xuzhou, Jiangsu 221300 (CN)**
• **CUI, Hushan
Xuzhou, Jiangsu 221300 (CN)**

• **CHENG, Shiran
Xuzhou, Jiangsu 221300 (CN)**
• **YU, Xiang
Xuzhou, Jiangsu 221300 (CN)**
• **ZOU, Zhiwen
Xuzhou, Jiangsu 221300 (CN)**
• **GUO, Delin
Xuzhou, Jiangsu 221300 (CN)**
• **HU, Dongdong
Xuzhou, Jiangsu 221300 (CN)**
• **XU, Kaidong
Xuzhou, Jiangsu 221300 (CN)**

(74) Representative: **Eisenführ Speiser
Patentanwälte Rechtsanwälte PartGmbB
Postfach 10 60 78
28060 Bremen (DE)**

(54) **ONLINE SAMPLER AND CONTAMINATION ANALYSIS SYSTEM**

(57)    An online sampler and a contamination analysis system. The online sampler is used for the contamination analysis system, and comprises a test tube module (200), a liquid taking tube (301), and a driving module (400); the test tube module (200) comprises a test tube (201), and the test tube (201) is used for receiving a scanning liquid dissolved with metal contamination; and the driving module (400) is used for driving the liquid taking tube (301) to ascend and descend relative to the test tube (201) to suction the scanning liquid in the test tube (201). The online sampler is suitable for the contamination analysis system integrating a collection system and a mass spectrometer into a whole, and can conveniently move the scanning liquid dissolved with the metal contamination from the collection system to the mass spectrometer to implement online measurement of the metal contamination.

Figure 3

# EP 4 372 355 A1

## Description

The present application claims priority to Chinese Patent Application No. 202110809061.2, titled "ONLINE SAMPLER AND SYSTEM FOR ANALYZING CONTAMINANT", filed on July 16, 2021 with the China National Intellectual Property Administration, which is incorporated herein by reference in its entirety.

## FIELD

The present disclosure relates to the field of semiconductor technology, and in particular to an online sampler and a system for analyzing contaminants.

## BACKGROUND

The semiconductor manufacture industry that develops gradually has set a goal for higher operating speed and smaller dimensions of devices, and a density of elements on a chip is increasing. Various chemical elements are kept being introduced in each generation of techniques, so as to improve a performance of the chips. Such introduction renders metallic contamination difficult to control in chip manufacturing. Thereby, it is necessary to detect metallic contaminants in manufacture, in order to ensure a throughput yield of devices such as chips.

At present, a system for collecting metallic contaminants using vapor phase decomposition (VPD) is combined with a mass spectrometer, such as an inductively coupled plasma mass spectrometer (ICPMS), for conventional detection on metallic contaminants. The collection system collects the metallic contaminants on a surface of a wafer, which serves as a target of analysis, using a scanning fluid in which the metallic contaminants are dissolved, and then transfers the scanning fluid having the dissolved metallic contaminants to the mass spectrometer for analysis.

In practice, it is desirable to integrate the collection system and the mass spectrometer for online analysis. Hence, an online sampler is needed to facilitate transferring the scanning fluid having the dissolved metallic contaminants from the collection system to the mass spectrometer.

## SUMMARY

An online sampler and a system for analyzing contaminants are provided according to embodiments of the present disclosure. The online sampler is applicable to the system for analyzing contaminants in which a collection system and a mass spectrometer are integrated, and is capable to move a scanning fluid having dissolved metallic contaminants from the collection system to the mass spectrometer. Hence, online measurement of metallic contaminants can be implemented.

In order to address at least the above technical issue, an online sampler is provided according to an embodiment of the present disclosure. The online sampler is applicable to a system for analyzing contaminants, and comprises a test-tube module, a fluid imbibing tube, and a driving module, where the test-tube module comprises a test tube configured to receive a scanning fluid having dissolved metallic contaminant, the driving module is configured to drive the fluid imbibing tube to move vertically with respect to the test tube to enable the fluid imbibing tube to imbibe the scanning fluid in the test tube.

In an embodiment, the online sampler further includes a driving part and a rinsing part. The driving part is configured to drive the test-tube module to rotate to switch the test tube between at least two positions, where a mouth of the test tube faces upwards when the test tube is at a first position of the at least two positions, and the mouth of the test tube faces downwards when the test tube is at a second position of the at least two positions. The rinsing part is configured to spout a rinsing fluid into the test tube when the mouth faces downwards.

In an embodiment, the rinsing part comprises a tank and a rinsing nozzle mounted at a bottom of the tank. The rinsing nozzle is connected to a source of the rinsing fluid, and is configured to spout the rinsing fluid. The tank is configured to collect a waste liquid of rinsing and the scanning fluid which is not imbibed from the test tube.

In an embodiment, the online sampler further comprises a drying module. The test tube is capable to be switched to a third position under driving of the driving part, and the drying module is configured to blow a drying gas into the test tube when the test tube is at the third position.

In an embodiment, the test-tube module comprises at least two test tubes, which are arranged in a same plane and surrounds a rotation center of the test-tube module, and the rotation center is aligned with a central axis of each of the at least two test tubes. A mouth of each of the at least two test tubes is disposed at a side of a bottom of said test tube away from the rotation center.

In an embodiment, the at least two test tubes are grouped into one or more test-tube pairs, and each of the one or more test-tube pairs comprises two of the at least two test tubes of which mouths face toward opposite directions.

In an embodiment, the test-tube module further comprises a carrier disk on which the test tube is mounted, and the carrier disk is fixedly connected to a rotation shaft of the driving part.

In an embodiment, the online sampler further comprises a rinsing module configured to rinse the fluid imbibing tube. The driving module is further configured to drive the fluid imbibing tube to switch between an imbibing position and a rinsing position, where the fluid imbibing tube is configured to coordinate with the test tube when being in the imbibing position and coordinate with the rinsing module when being in the rinsing position. The driving module is further configured to drive the fluid imbibing tube to move vertically with respect to the rinsing module.

In an embodiment, the rinsing module comprises at least one rinsing unit. Each of the at least one rinsing unit comprises a rinsing body having a rinsing chamber, and an inlet pipe and an outlet pipe, each of which connects the rinsing chamber. The inlet pipe is configured to inject another rinsing fluid into the rinsing chamber, and the outlet pipe is configured to drain the another rinsing fluid. The inlet pipe is provided with an inlet valve, and the outlet pipe is provided with an outlet valve.

In an embodiment, each of the at least one rinsing units further comprises a liquid-level sensor mounted on the rinsing body.

In an embodiment, the rinsing body comprises a connector mounted at a top of the rinsing chamber, and the connector is connected to an overflow pipe.

In an embodiment, the fluid imbibing tube is mounted on a swing arm, and the driving module comprises a lift-driving component and a rotation-driving component mounted on a part of the lift-driving component capable to move vertically. The swing arm is connected to an action terminal of the rotation-driving component. The rotation-driving component is configured to drive the swing arm to rotate to switch the fluid imbibing tube between the imbibing position and the rinsing position.

In an embodiment, the online sampler further comprises a volume acquiring module configured to determine a volume of the scanning fluid in the test tube.

In an embodiment, the volume acquiring module comprises a light source, an image capturing device, and an image analyzer. The light source and the image capturing device are disposed at two sides, respectively, of the test tube. The image capturing device is configured to capture an image of the test tube containing the scanning fluid and transmit the image to the image analyzer. The image analyzer is configured to determine the volume of the scanning fluid in the test tube based on the image.

In an embodiment, the test tube comprises a large-diameter section and a small-diameter section which are connected, and a mouth of the test tube is located at the large-diameter section.

In an embodiment, the online sampler further comprises a sampling box having a chamber, and the test-tube module and the volume acquiring module are disposed in the chamber.

In an embodiment, the sampling box is further provided with an air pumping port connected to the chamber.

A system for analyzing contaminants is further provided according to an embodiment of the present disclosure. The system comprises a collection system and a mass spectrometer that are integrated, and any foregoing online sampler. A scanning-fluid nozzle of the collection system is located at a position corresponding to the test-tube module. An end of the fluid imbibing tube is configured to imbibe a liquid from the test tube, and another end of the fluid imbibing tube is connected to the mass spectrometer.

In an embodiment, the collection system is configured to collect metallic contaminants through vapor phase decomposition, and/or the mass spectrometer is an inductively coupled plasma mass spectrometer.

The online sampler according to the present disclosure is applicable to the system for analyzing contaminants in which the collection system and the mass spectrometer are integrated. During operation, the online sampler may be located at a fixed position with respect to the collection system, that is, the online sampler and the collection system may also be integrated. The test tube in the test-tube module of the online sampler may be configured to contain the scanning fluid having dissolved metallic contaminants, which is spouted from the scanning-fluid nozzle of the collection system. The fluid imbibing tube is driven by the driver module to move vertically, so that the fluid imbibing tube dips into the test tube to imbibe the scanning fluid in the test tube and transfers the scanning fluid to the mass spectrometer. Accordingly, the mass spectrometer can analyze the metallic contaminants in the scanning fluid. Such structure of the online sampler is capable to transfer the scanning fluid, which has the dissolved metallic contaminants and is collected by the collection system, to the mass spectrometer conveniently, and thereby implements online measurement of the metallic contaminants and improves efficiency of detecting the metallic contaminants.

In one embodiment, the online sampler is provided with the driving part and the rinsing part. Under the driving of the driving part, the test tube in the test-tube module can be switched between the first position at which its mouth faces upwards and the second position at which its mouth faces downwards. The test tube can receive the scanning fluid when being at the first position, and can be rinsed by the rinsing part, which spouts the rinsing fluid into the test tube, when being at the second position. Thereby, the online sampler can rinse the test tube and protect obtained scanning-fluid samples from cross contamination, which facilitates automation of online sampling.

In an embodiment, the online sampler is further provided with the drying module. Under the driving of the driving part, the test tube in the test-tube module can be switched further to the third position. When the test tube is at the third position, the drying module is capable to blow the drying gas into the test tube, so that the test tube can be dried after being rinsed. Thereby, it is prevented that the remaining rinsing fluid affects detection accuracy, and duration of drying the rinsed test tube is shortened. Detection efficiency is improved.

In an embodiment, the online sampler is further provided with the rinsing module for rinsing the fluid imbibing tube. The driving module can drive the fluid imbibing tube to be switched between the imbibing position at which the fluid imbibing tube coordinates with the test tube and the rinsing position at which the fluid imbibing tube coordinates with the rinsing module. Thereby, the scanning-fluid samples, which are transferred by the fluid imbibing tube to the mass spectrometer, can be protected from cross contamination. Detection accuracy is improved.

In an embodiment, the online sampler is further provided with the volume acquiring module for determining the volume of the scanning fluid in the test tube. The volume acquiring module provides a basis for precise analysis on the metallic contaminants.

## BRIEF DESCRIPTION OF THE DRAWINGS

Figure 1 is a schematic structural diagram of an online sampler according to an embodiment of the present disclosure.

Figure 2 is a schematic diagram of an internal structure of an online sampler according to an embodiment of the present disclosure.

Figure 3 is a schematic cross-sectional diagram of an online sampler according to an embodiment of the present disclosure.

Figure 4 is a schematic structural diagram of coordination between a test-tube module and a driving part as shown in Figure 3 according to an embodiment of the present disclosure.

Figure 5 is a schematic structural diagram of a test tube of a test-tube module at a rinsing position according to an embodiment of the present disclosure.

Figure 6 is a schematic structural diagram of a test tube of a test-tube module at a drying position according to an embodiment of the present disclosure.

Figure 7 is a schematic structural diagram of a test tube containing a scanning fluid according to an embodiment of the present disclosure.

Figure 8 is a schematic structural diagram of coordination between a fluid imbibing tube and a driving module according to an embodiment of the present disclosure.

Figure 9 is a schematic structural diagram of a rinsing unit of a rinsing module according to an embodiment of the present disclosure.

Reference numerals:

| | | | |
|---|---|---|---|
| sampling box | 100, | bottom wall | 101, |
| top wall | 102, | side wall | 103, |
| test-tube module | 200, | test tube | 201, |
| large-diameter section | 211, | small-diameter section | 212, |
| transition portion | 213, | carrier disk | 202, |
| fluid imbibing tube | 301, | swing arm | 302, |
| first arm | 321, | second arm | 322, |
| driving module | 400, | sliding platform | 401, |
| rotation-driving component | 402, | coupler | 421, |
| driving part | 500, | rinsing part | 600, |

(continued)

| tank | 601, | rinsing nozzle | 602, |
|---|---|---|---|
| draining port | 603, | drying module | 700, |
| gas nozzle | 701, | rinsing module | 800, |
| rinsing body | 801, | rinsing chamber | 811, |
| inlet pipe | 802, | inlet valve | 821, |
| outlet pipe | 803, | outlet pipe | 831, |
| liquid-level sensor | 804, | first connector | 805, |
| second connector | 806, | overflow pipe | 807, |
| volume acquiring module | 900, | light source | 901, |
| image capturing device | 902. | | |

## DETAILED DESCRIPTION OF EMBODIMENTS

Hereinafter the present disclosure will be further described in detail in conjunction with the drawings and specific embodiments such that those skilled in the art understand the present disclosure better.

Hereinafter a system for analyzing contaminants and an online sampler would be illustrated in combination for the sake of appreciation and conciseness, and beneficial effects would not be repeated.

The system for analyzing contaminants according to embodiments of the present disclosure is applicable to detection and analysis of metallic contaminants in manufacturing of semiconductor elements such as wafers. The system comprises a collection system and a mass spectrometer that are integrated. The collection system is configured to collect metallic contaminants on a surface of a wafer, which serves as a target of analysis, using a scanning fluid, so that the metallic contaminants are dissolved in the scanning fluid. The mass spectrometer is configured to analyze the scanning fluid having the dissolved metallic contaminants. The online sampler according to embodiments of the present disclosure can transfer the scanning fluid having the dissolved metallic contaminants directly to the mass spectrometer for timely detection. Adverse phenomena, such as being polluted, are prevented from affecting detection results during the transfer. Hereinafter the scanning fluid having dissolved metallic contaminants is called a scanning-fluid sample to facilitate description.

The collection system may be a system for collecting metallic contaminants using vapor phase decomposition, and the mass spectrometer may be an inductively coupled plasma mass spectrometer. It is appreciated that the collection system and the mass spectrometer both may be in other forms. An emphasis of embodiments of the present disclosure lies in a structural design of the online sampler. Hereinafter a structure is described in conjunction with the drawings.

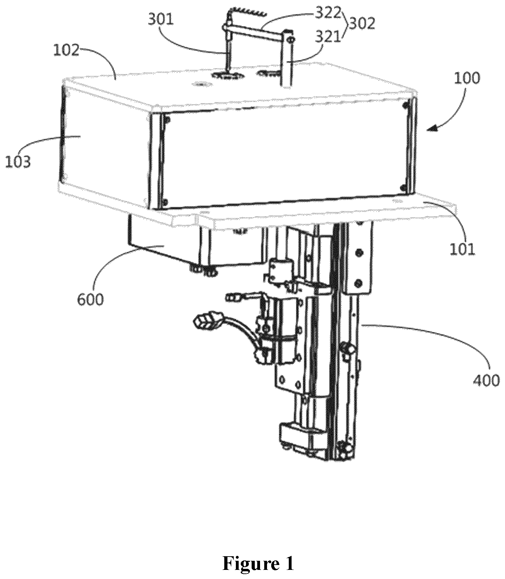

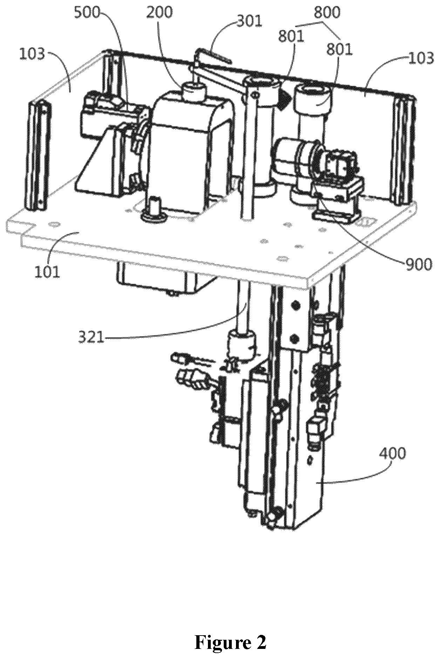

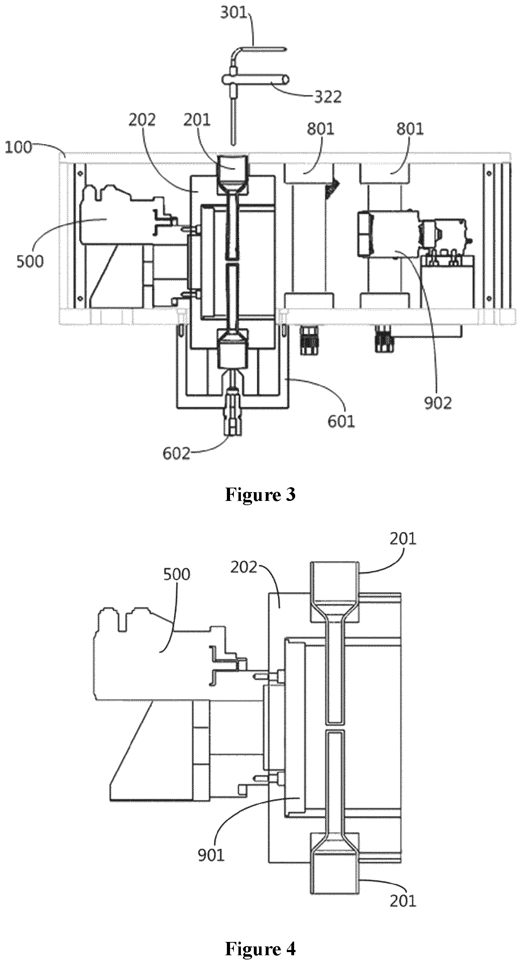

Reference is made to Figures 1 to 4. Figure 1 is a schematic structural diagram of an online sampler according to an embodiment of the present disclosure. Figure 2 is a schematic diagram of an internal structure of an online sampler according to an embodiment of the present disclosure. Figure 3 is a schematic cross-sectional view of an online sampler according to an embodiment of the present disclosure. Figure 4 is a schematic structural diagram of a test-tube module and a driving part, as shown in Figure 3, according to an embodiment of the present disclosure.

Herein the online sampler comprises a test-tube module 200, a fluid imbibing tube 301, and a driving module 400. The test-tube module 200 comprises a test tube 201, which is configured to receive a scanning-fluid sample. The driver module 400 is configured to drive the fluid imbibing tube 301 to move vertically with respect to the test tube 201, so that the fluid imbibing tube 301 is capable to imbibe the scanning-fluid sample in the test tube 201. That is, the fluid imbibing tube 301 may descend relative to the test tube 201, so as to dip into the test tube 201 to imbibe a liquid, and may ascend relative to the test tube 201, so as to move out of the test tube 201.

In practice, the online sampler and the collection system may be integrated, that is, may be fixed relative to each other, such that a position of a scanning-fluid nozzle of the collection system for collecting the scanning-fluid sample corresponds to that of the test tube 201. Thereby, the scanning-fluid nozzle of the collection system may spout the scanning-fluid sample directly into the test tube 201. An end of the fluid imbibing tube 301 is configured to imbibe a liquid from the test tube 201, and another end of the fluid imbibing tube 301 may be connected to a corresponding pipeline of the mass spectrometer, so as to transfer the scanning-fluid sample imbibed from the test tube 201 to the mass spectrometer.

The above configuration of the online sampler provides a basis for online measurement of the system for analyzing contaminants, and can transfer the scanning-fluid sample collected by the collection system conveniently to the mass spectrometer. Not only less time is consumed by the transfer, but also the scanning-fluid sample is less likely to be polluted during the transfer. Efficiency and accuracy of detection are improved.

The system for analyzing contaminants may further comprise a control module (not depicted). The control module is connected to the collection system, the mass spectrometer, and the online sampler. The control module controls the scanning-fluid nozzle of the collection system to spout the scanning-fluid sample into the test tube 201, controls the fluid imbibing tube 301 to imbibe a liquid from the test tube 201 and transfer the liquid to the mass spectrometer, controls a rinsing module 800 to rinse the fluid imbibing tube 301, and controls rinsing of the test-tube module 200. Thereby, full-automation of the system may be implemented without manual operation of an operator, which improves operation efficiency.

In the embodiment, the online sampler further comprises a driving part 500 and a rinsing part 600. The driving part is configured to drive the test-tube module 200 to rotate, so as to switch the test tube 201 between at least two positions. When the test tube 201 is at a first position, a mouth of the test tube 201 faces upwards. In such case, the test tube 201 is in a state capable of receiving the scanning-fluid sample, and the scanning-fluid sample can be imbibed by the fluid imbibing tube 301. When the test tube 201 is at a second position, the mouth of the test tube 201 faces downwards, and the rinsing part 600 is capable to spout a rinsing fluid into the test tube 201 via the mouth to rinse the test tube 201. When being at the second position, the test tube 201 is actually in a state capable of being rinsed.

The mouth of the test tube 201 facing upwards at the first position or facing downwards at the second position does not limit that the test tube 201 must be disposed strictly along a vertical direction. That is, the test tube 201 may be inclined with its mouth upwards or downwards, as long as a requirement of the corresponding position is met. In actual arrangement, it is preferable that the test tube 201 is disposed along a vertical direction at the first position and the second position. That is, it is preferable that the mouth of the test tube 201 faces vertically upwards at the first position, so as to facilitate measure on a volume of the scanning-fluid sample in the test tube 201 on a requirement (which would be described later). Moreover, it is preferable that the test tube 201 faces vertically downwards at the second position, so as to facilitate the rinsing fluid spouted into the test tube 201 flowing out of the test tube 201 and hence minimizing its residue in the test tube 201.

In a case that the driving part 500 and the rinsing part 600 are provided as above, the position of the test tube 201 may be switched by the driving part 500 to facilitate rinsing of the test tube 201. Thereby, cross contamination among different scanning-fluid samples is avoided, the online sampling is more automated, and efficiency of detection is improved.

The online sampler may be further provided with a drying module 700, such that the rinsed test tube 201 can be quickly dried, which reduces an impact of the residual rinsing fluid on detection of a next scanning-fluid sample. The driving part 500 may be further configured to drive the test tube 201 to be switched to a third position. When the test tube 201 is at the third position, the drying module 700 can blow a drying gas into the test tube 201. In practice, the test tube 201 is disposed at the first position first to receive the scanning-fluid sample. After the scanning-fluid sample is imbibed, the driving part 500 drives the test tube 201 to be rotated to the second position, and the rinsing part 600 rinses the test tube 201. Then, the driving part 500 drives the test tube 201 to be rotated to the third position, and the test tube 201 is dried by the drying module 700. After the drying, the driving part 500 drives the test tube 201 to be rotated back to the first position, so that the test tube 201 can receive a next scanning-fluid sample. The above cycle is repeated.

Both the rinsing part 600 and the drying module 700 are disposed along a rotation trajectory of the test tube 201, such that the test tube 201 can be rinsed and dried.

In an embodiment, the test tube 201 is disposed along a vertical direction at the first position and the second position, in order to facilitate measurement and arrangement. The rinsing part 600 for rinsing the test tube 201 may comprise a tank 601 and a rinsing nozzle 602 disposed at the bottom of the tank 601. The rinsing nozzle 602 is connected to a source of a rinsing fluid (not depicted), and is configured to spout the rinsing fluid into the test tube 201, which is at the second position, so as to rinse the test tube 201. The tank 601 is configured to collect a waste rinsing liquid after the test tube 201 is rinsed. Generally, the rinsing fluid is ultra-pure water (UPW).

In practice, the fluid imbibing tube 301 is only required to imbibe and transfer only a part of the scanning-fluid sample from the test tube 201 to the mass spectrometer. The remaining scanning-fluid sample in the test tube 201 that has not been imbibed may be poured into the tank 601 when the test tube 201 into the second position, and thus is collected by the tank 601.

In the embodiment, the test-tube module 200 comprises at least two test tubes 201, which are arranged in a same plane and surrounds a rotation center of the test-tube module 200. The rotation center is located on a central axis of each test tube 201. A mouth of the test tube 201 is located at a side of a bottom of such test tube 201 away from the rotation center. Thereby, since the at least two test tubes 201 are provided and the test-tube module 200 is rotated under driving of the driving part 500, all test tubes 201 are sequentially disposed at the first position for receiving the scanning-fluid sample. Different test tubes 201 may receive different scanning-fluid samples, thereby improving efficiency of detection.

Since the test tube 201 has a certain volume, arranging the test tubes 201 of the test-tube module 200 in the same planer refers to that the central axis of the test tubes 201 are disposed in such plane. Thereby, when the test-tube module 200 rotates, the first position, the second position, and the third position each is ensured to be identical among the test tubes 201, which facilitates determining a position of the online sampler relative to the collection system and determining positions of the rinsing part 600 and the drying module 700 of the online sampler.

In an embodiment as shown in the figures, it is taken as an example that the test-tube module 200 comprises two test tubes 201. The two test tubes 201 form a test-tube pair, and mouths of the two test tubes 201 face opposite directions. That is, the central axes of the two test tubes 201 are aligned along a straight line.

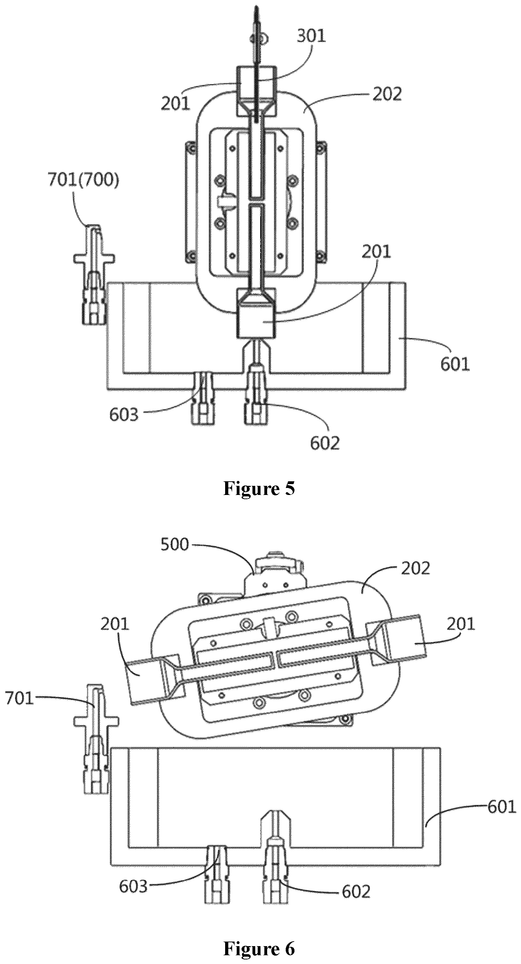

Reference is made to Figure 5 in combination with Figure 6. Figure 5 is a schematic structural diagram a test tube of a test-tube module at a second position according to an embodiment of the present disclosure. Figure 6 is a schematic structural diagram of a test tube of a test-tube module at a third position according to an embodiment of the present disclosure.

The test-tube module 200 as shown in Figures 5 and 6 may be regarded as a right view corresponding to that as shown Figure 4. Generally, the driving part 500 comprises a motor, and may alternatively be another component capable to drive rotation. A rotation shaft of the motor is connected to the test-tube module 200, so as to drive rotation of the test-tube module 200. In an embodiment, the test-tube module 200 comprises a carrier disk 202, and each test tube 201 is mounted on the carrier disk 202. The carrier disk 202 is fixedly connected to the rotation shaft of the driving part 500.

In the view as shown in Figure 4, an axis of the rotation of the test-tube module 200 is along a horizontal direction in the paper. In the view as shown in Figures 5 and 6, the test tubes 201 of the test-tube module 200 are rotated within in a plane of paper.

In an example as shown in Figure 5, the test tube 201 at an upper position is located at the first position, and is capable to receive the scanning-fluid sample. In such case, the fluid imbibing tube 301 is capable to imbibe and transfer the scanning-fluid sample from the test tube 201 to the mass spectrometer. The test tube 201 at a lower position is located at the second position. The structure is such configured that when the test tube 201 is at the second position, the test tube 201 dips into the tank 601 and corresponds to a position of the rinsing nozzle 602 at the bottom of the tank 601. Thereby, the rinsing nozzle 602 is capable to spout the rinsing fluid into the test tube 201 to rinse the test tube 201. Relative arrangement between or structures of the tank 601 and the test-tube module 200 shall not affect the rotation of the test-tube module 200.

In an embodiment, a draining port 603 is further provided at the bottom of the tank 601 in the rinsing part 600. The draining port 603 may be connected to a pipe via a pipe joint to drain a waste liquid from the tank 601.

In an embodiment, the drying module 700 comprises a gas nozzle 701 and a source of the drying gas (not depicted). The gas nozzle 701 may be mounted at a side of the tank 601 of the rinsing part 600, so as to simplify the structure.

After the test tube 201 at the lower position is rinsed at the second position as shown in Figure 5, the test-tube module 200 may rotate clockwise by a certain angle under driving of the driving part 500 to a state as shown in Figure 6. At such time, the test tube 201 previously at the lower position is rotated to a position corresponding to the gas nozzle 701, that is, to the third position, and the gas nozzle 701 blows drying gas into the test tube 201 to dry the test tube 201. At such time, the test tube 201 previously at the upper position as shown in Figure 5 is rotated to a right side as shown in Figure 6. As shown in Figure 6, after the nozzle 701 dries the left test tube 201, the driving part 500 drives the test-tube module 200 to rotate again, so that dried the test tube 201 moves to the first position and the right test tube 201 moves to the second position, i.e., a position for rinsing. Thereby, each time one test tube 201 in the test-tube pair is located at the first position for receiving the scanning-fluid sample, the other test tube 201 in the test-tube pair is located at the second position and hence can be rinsed.

In practice, there may be multiple forgoing test-tube pairs, so as to improve efficiency of utilizing the online sampler and shorten sampling time.

An angle by which the test tube 201 is rotated from the second position to the third position may be set according to an actual requirement, as long as it is convenient for the gas nozzle 701 to blow the drying gas into the test tube 201 being disposed at the third position.

In practice, the drying gas utilized by the drying module for drying the test tube 201 may be nitrogen. IN an embodiment, the nitrogen may be ultrapure nitrogen (of which purity is greater than 99.999%), so as to prevent the dried test tube 201 from being contaminated by residual impurities which affect a result of detecting metallic contaminants. Temperature of the nitrogen may exceed 100°C to ensure an effect drying the test tube 201. The drying gas may be another gas not affecting cleanliness of the test tube 201.

In practice, a quantity of spouts from the rinsing nozzle 602 may be controlled each time the test tube 201 is rinsed, so as to ensure that the rinsed test tube 201 is clean. For example, the rinsing liquid may be spouted for 1 to 10 times within 1 to 5 seconds. A quantity of blows of the gas nozzle 701 may further be controlled each time the test tube 201 is dried, so as to ensure that the test tube 201 is completely dried. For example, the drying gas may be blown for 1 to 10 times within 1 to 5 seconds.

In an embodiment, the online sampler is further provided with a rinsing module 800 configured to rinse the fluid imbibing tube 301. Each time the fluid imbibing tube 301 imbibes a liquid, it would be rinsed by the rinsing module 800 to avoid cross contamination among the scanning-fluid samples. The driving module 400 may be further configured to drive the fluid imbibing tube 301 to move, so as to facilitate the fluid imbibing tube 301 imbibing the liquid from the test tube 201 and being rinsed by the rinsing module 800. Under such driving, the fluid imbibing tube 301 may be switched between an imbibing position, at which it coordinates with the test tube 201, and a rinsing position, at which it coordinates with the rinsing module 800. The driving module 400 may be further configured to drive the fluid imbibing tube 301 to move vertically with respect to the rinsing module 800.

In one embodiment, the fluid imbibing tube 301 rotates under the driving, so as to switch between the imbibing position and the rinsing position, which facilitates configuration of the driving module 400.

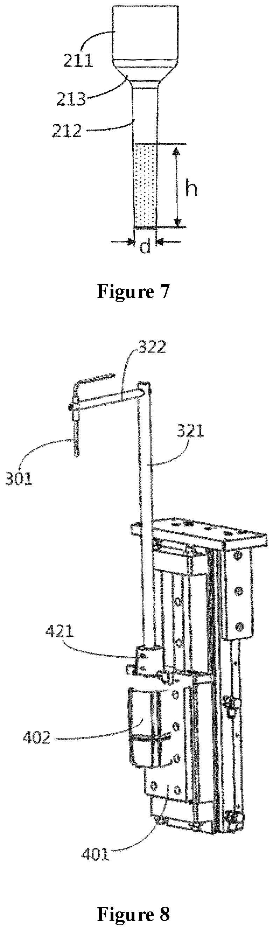

Reference is made to Figure 8, which is a schematic structural diagram of coordination of the fluid imbibing tube and the driving module according to an embodiment of the present disclosure.

In an embodiment, the fluid imbibing tube 301 is mounted on a swing arm 302 which is connected to the driving module 400.

The driving module 400 may comprise a lift-driving component and a rotation-driving component 402. In an embodiment, the lift-driving component may comprise a source for driving lifting motion, such as a telescopic cylinder or an electric actuator. A sliding platform 401 may be fixedly connected to a lifting part of such source, and the rotation-driving component 402 is mounted on the sliding platform 401. An action terminal of the rotation-driving component 402 is connected to the swing arm 302. Thereby the rotation-driving component 402, the swing arm 302, and the fluid imbibing tube 301 can move vertically together under driving of the lift-driving component. The lift-driving component may drive the fluid imbibing tube 301 to move vertically within a range from 0mm to 50mm. The rotation-driving component 402 may drive the swing arm 302 to rotate and thereby drive the fluid imbibing tube 301 to rotate. Thereby, the fluid imbibing tube 301 may rotate within a range from 0 degree to 360 degree.

The lift-driving component may cooperate with a guiding part, which is configured to guide the vertical movement of the sliding platform 401. The sliding platform 401 is mounted and capable to slide on the guiding part.

In an embodiment, the swing arm 302 comprises a first arm 321 extending vertically and a second arm 322 perpendicular to the first arm 321. The fluid imbibing tube 301 is mounted at an end of the second arm 322. The first arm 321 is connected to the rotation-driving component 402. The rotation-driving component 402 may be a component such as a motor, and a driving shaft of the motor may be connected to the first arm 321 via a coupler 421. The fluid imbibing tube 301 may rotate with respect to a central axis of the first arm 321, and a length of the second arm 322 may serve as a radius of rotation of the fluid imbibing tube 301. The first position for the test tube 201 and the rinsing module 800 shall be position at a trajectory of rotation of the fluid imbibing tube 301.

When the fluid imbibing tube 301 is required to imbibe a liquid, a height of the fluid imbibing tube 301 with respect to the test tube 201 is adjusted by the lift-driving component. The driving component 402 drives the fluid imbibing tube 301 to rotate to the imbibing position corresponding to the test tube 201. Then, the lift-driving component drives the fluid imbibing tube 301 to descend and dip into the test tube 201 for imbibing the scanning-fluid sample. Afterwards, the lift-driving component drives the fluid imbibing tube 301 to ascend out of the test tube 201. The driving component 402 then drives the fluid imbibing tube 301 to rotate to the rinsing position for rinsing.

In an embodiment, the rinsing module 800 comprises at least one rinsing unit. As shown in Figures 2 and 3, it is taken as an example that the rinsing module 800 comprises two rinsing units. The rinsing fluids in the two rinsing units may be different, so that the fluid imbibing tube 301 may be rinsed twice successively to ensure cleanliness of the fluid imbibing tube 301. It is appreciated that the fluid imbibing tube 301 may rinsed only once, or more rinsing units may be provided. Specific configuration may be determined according to an actual requirement. The rinsing units may be substantially identical in structure.

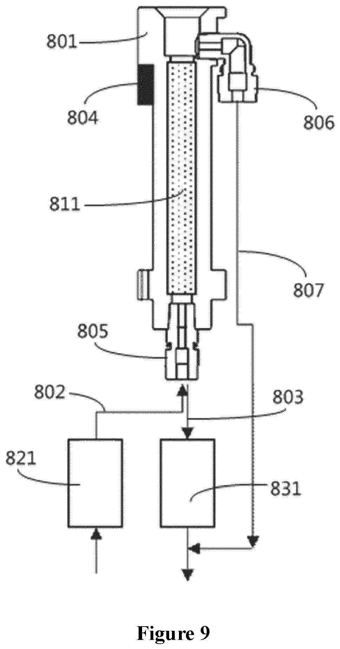

Reference is made to Figure 9, which is a schematic structural diagram of a rinsing unit of a rinsing module according to an embodiment of the present disclosure.

In an embodiment, the rinsing unit comprises a rinsing body 801. The rinsing body 801 has a rinsing chamber 811, which is configured to accommodate the rinsing fluid. The rinsing unit further comprises an inlet pipe 802 and an outlet pipe 803, both of which connect the rinsing chamber 811. The inlet pipe 802 is configured to inject the rinsing fluid into the rinsing chamber 811, and the outlet pipe 803 is configured to eject the rinsing fluid. The inlet pipe 802 and the outlet pipe 803 may be connected to the rinsing chamber 811 via the same port, so as to simplify the structure. The inlet pipe 802 is provided with an inlet valve 821, and the inlet pipe 802 is opened and shut under control of the inlet valve 821. The outlet pipe 803 is provided with an outlet valve 831, and the outlet pipe 803 is opened and shut under control of the outlet valve 831.

As shown in Figure 9, the port is provided at a bottom of the rinsing body 811 and connects with the rinsing chamber 811, which simplifies the structure. A first connector 805 is mounted on the port, and the inlet pipe 802 and the outlet pipe 803 are connected to the first connector 805.

The fluid imbibing tube 301 is inserted into the rinsing chamber 811 of the rinsing body 801 for rinsing. When being at the rinsing position, the fluid imbibing tube 301 is driven by the lift-driving component to move vertically, so as to dip into the rinsing chamber 811 or ascend out of the rinsing chamber 811.

In order to the fluid imbibing tube 301 dipping into the rinsing chamber 811 of the rinsing body 801, an opening is provided at a top of the rinsing body 801 to expose the rinsing chamber 811.

In an embodiment, the rinsing unit may further comprise a liquid-level sensor 804, which is disposed at an upper part of the rinsing body 811 close to the rinsing chamber 811. When the rinsing fluid is injected into the rinsing chamber 811, the liquid-level sensor 804 may detect that the liquid-level of the rinsing fluid reaches a set value and thereby the inlet valve 821 can be closed in time, so as to prevent the rinsing fluid from overflowing from the rinsing body 801.

In an embodiment, the rinsing body 811 may be provided with an overflow port connecting the rinsing chamber 811 at an upper part of the rinsing chamber 811. A second connector 806 is mounted at the overflow port, and the second connector 806 is connected to an overflow pipe 807. In a case that the liquid-level sensor 804 fails or is insensitive, the rinsing fluid can be flows via the second connector 806 and the overflow pipe 807 when the rinsing chamber 811 has been filled with the rinsing fluid. The overflow pipe 807 may be connected to the outlet pipe 803. It is appreciated that the outlet valve 831 is located on the outlet pipe 803 at a position between the port of the rinsing chamber 811 and a joint between the overflow pipe 807 and the outlet pipe 803.

In an embodiment, the overflow pipe 807 may a perfluoroalkoxy (PFA) pipe.

Accurate measurement on a volume of the scanning-fluid sample is also a foundation of the analysis on metallic contaminants. In an embodiment, the online sampler further comprises a volume acquiring module 900. The volume acquiring module 900 is configured to determine a volume of the scanning-fluid sample in the test tube 201.

In an embodiment, the volume acquiring module 900 may adopt optical measurement, so as to simplify the structure. Reference is made to Figures 2, 3, and 4. The volume acquiring module 900 comprises a light source 901, an image capturing device 902, and an image analyzer. The image capturing device 902 and the light source 901 are disposed at two sides, respectively, of the test tube 201. The image capturing device 902 is configured to capture an image of the test tube 201 containing the scanning-fluid sample and transmit the image to the image analyzer. The image analyzer is configured to determine the volume of the scanning-fluid sample in the test tube 201 based on such image. The light source 901 is configured to provide sufficient illumination for the capture of the image. The image capturing device 902 may be a conventional camera, or the like. In an embodiment, a camera with a pixel quantity ranging from 2 million to 10 million may be selected.

The light source 901 may be mounted on the carrier disk 202, that is, the light source 901 and the test tube 201 may be rotated together, so as to simplify the structure and save a space.

In an embodiment, the image analyzer is configured to determine a liquid level of the scanning-fluid sample in the test tube 201 based on the acquired image. An inner diameter of the test tube 201 may be determined in advance, such that the volume of the scanning-fluid sample in the test tube 201 can be determined accordingly.

Generally, the scanning-fluid sample of a small volume is spouted into the test tube 201 by the scanning-fluid nozzle of the collection system. The test tube 201 may have a shape similar to a stick grenade, as shown in Figure 7, so as to facilitate measurement and reception of the scanning-fluid sample. The test tube 20 comprises a large-diameter section 211 and a small-diameter section 212 which are connected. The mouth of the test tube 20 is located in the large-diameter section 211 to facilitate the scanning-fluid nozzle of the collection system spouting the scanning-fluid sample into the tube body 211. After being spouted, the scanning-fluid sample falls into the small-diameter section 212 that facilitates measurement. In practice, a diameter-to-length ratio of the small-diameter section 212 may be set according to an actual requirement for facilitating measurement.

In an embodiment, the test tube 201 further comprises a transition portion 213 between the large-diameter section 211 and the small-diameter section 212. The transition portion 213 has a conical structure, that is, a radial dimension of the transition portion 213 gradually decreases from the large-diameter section 211 to the small-diameter section 212. Hence, it can be ensured that the scanning-fluid sample spouted into the test tube 201 falls thoroughly into the small-diameter section 212.

As shown in Figure 7, the inner diameter d of the small-diameter section 212 of the test tube 201 may be predetermined. After the liquid level *h* of the scanning-fluid sample is determined by the imaging analyzer, the volume

$$V = \pi * \left(\frac{d}{2}\right)^2 * h$$

of the scanning-fluid sample in the scanning tube 201 can be determined through .

Reference is made to Figures 1 and 2. In an embodiment, the online sampler comprises a sampling box 100, which facilitates installation and arrangement. The above structures may be disposed in the sampling box 100. In an embodiment, the sampling box 100 has a chamber, and the test-tube module 200, the driving part 500, the rinsing module 800, and the volume acquiring module 900 may be mounted in the chamber. Thereby, the components are protected from external interferences, especially that on the volume acquiring module 900 due to ambient light.

In an embodiment, the sampling box 100 comprises a bottom wall 101 and a top wall 102 opposite to each other, and further comprises sidewalls 103 forming an enclosure between the bottom wall 101 and the top wall 102. Four side walls 103 are depicted in the figures, and the components may be mounted on the bottom wall 101 via a support or the like. The tank 601 of the rinsing part 600 may be mounted outside the chamber at a lower part of the bottom wall 101. Correspondingly, an opening may be formed at the bottom wall 101, and the first connector 805 of the rinsing unit extends out of the bottom wall 101 to facilitate connection of relevant pipes.

Another opening may be provided at an appropriate position of the sampling box 100 based on a requirement of mounting another structure, and the details are not repeated herein.

In an embodiment, the sampling box 100 may be further provided with an air pumping port. The air pumping port may be connected to a venting pipe of the collection system, so that the chamber of the sampling box 100 has a negative pressure state which prevents a corrosive gas or an impurity in the sampling box 100 from escaping. Draining structures of the online sampler, such as the draining port 603 of the tank 601 and the outlet pipe 803 of the rinsing unit, may be connected to a relevant draining pipe of the collection system. The foregoing pipe(s) may be provided with a one-way control mechanism for preventing pollutants from flowing back to the sampling box 100.

In practice, the test tube 201, the fluid imbibing tube 301, and the rinsing body 801 may be made of any one or any combination of: perfluoroalkoxy (PFA), polytetrafluoroethylene (PTFE), ethylene tetrafluoroethylene (ETFE), polychlorotrifluoroethylene ethylene (PCTFE), polyvinylidene fluoride (PVDF), polyvinyl chloride (PVC), polypropylene (PP), polyether ether ketone (PEEK), polyphenylene sulfide (PPS), and polymethyl methacrylate (PMMA).

Hereinabove the online sampler and the system for analyzing contaminants are illustrated in detail according to embodiments of the present disclosure. Specific embodiments have been used for illustrating principles and the implementations of the present disclosure, and the description thereof are only intended for helping understand means and core concepts of the present disclosure. Those skilled in the art may made several improvements and modifications to the present disclosure without departing from the principles of the present disclosure, and these improvements and modifications shall fall within the protection scope of the claims of the present disclosure.

## Claims

1. An online sampler, applicable to a system for analyzing contaminants, wherein the online sampler comprises:

   a test-tube module,
   a fluid imbibing tube, and
   a driving module, wherein:

      the test-tube module comprises a test tube configured to receive a scanning fluid having dissolved metallic contaminant, and
      the driving module is configured to drive the fluid imbibing tube to move vertically with respect to the test tube to enable the fluid imbibing tube to imbibe the scanning fluid in the test tube.

2. The online sampler according to claim 1, further comprising:

   a driving part, configured to drive the test-tube module to rotate to switch the test tube between at least two positions, wherein a mouth of the test tube faces upwards when the test tube is at a first position of the at least two positions, and the mouth of the test tube faces downwards when the test tube is at a second position of the at least two positions; and
   a rinsing part, configured to spout a rinsing fluid into the test tube when the mouth faces downwards.

3. The online sampler according to claim 2, wherein the rinsing part comprises:

   a tank, configured to collect a waste liquid of rinsing and the scanning fluid which is not imbibed from the test tube; and
   a rinsing nozzle, mounted at a bottom of the tank, wherein the rinsing nozzle is connected to a source of the rinsing fluid, and is configured to spout the rinsing fluid.

4. The online sampler according to claim 2, further comprising a drying module, wherein:

   the test tube is capable to be switched to a third position under driving of the driving part, and
   the drying module is configured to blow a drying gas into the test tube when the test tube is at the third position.

5. The online sampler according to claim 2, wherein:

   the test-tube module comprises at least two test tubes, which are arranged in a same plane and surrounds a rotation center of the test-tube module;

the rotation center is aligned with a central axis of each of the at least two test tubes; and

a mouth of each of the at least two test tubes is disposed at a side of a bottom of said test tube away from the rotation center.

6. The online sampler according to claim 5, wherein the at least two test tubes are grouped into one or more test-tube pairs, and each of the one or more test-tube pairs comprises two of the at least two test tubes of which mouths face toward opposite directions.

7. The online sampler according to any one of claims 2 to 6, wherein the test-tube module further comprises a carrier disk on which the test tube is mounted, and the carrier disk is fixedly connected to a rotation shaft of the driving part.

8. The online sampler according to any one of claims 1 to 6, further comprising:

   a rinsing module, configured to rinse the fluid imbibing tube, wherein:

   the driving module is further configured to drive the fluid imbibing tube to switch between an imbibing position and a rinsing position, wherein the fluid imbibing tube is configured to coordinate with the test tube when being in the imbibing position and coordinate with the rinsing module when being in the rinsing position; and

   the driving module is further configured to drive the fluid imbibing tube to move vertically with respect to the rinsing module.

9. The online sampler according to claim 8, wherein:

   the rinsing module comprises at least one rinsing unit;

   each of the at least one rinsing unit comprises a rinsing body having a rinsing chamber, and an inlet pipe and an outlet pipe, each of which connects the rinsing chamber;

   the inlet pipe is configured to inject another rinsing fluid into the rinsing chamber, and the outlet pipe is configured to drain the another rinsing fluid; and

   the inlet pipe is provided with an inlet valve, and the outlet pipe is provided with an outlet valve.

10. The online sampler according to claim 9, wherein each of the at least one rinsing units further comprises a liquid-level sensor mounted on the rinsing body.

11. The online sampler according to claim 10, wherein the rinsing body comprises a connector mounted at a top of the rinsing chamber, and the connector is connected to an overflow pipe.

12. The online sampler according to claim 8, wherein:

   the fluid imbibing tube is mounted on a swing arm;

   the driving module comprises a lift-driving component and a rotation-driving component mounted on a part of the lift-driving component capable to move vertically;

   the swing arm is connected to an action terminal of the rotation-driving component; and

   the rotation-driving component is configured to drive the swing arm to rotate to switch the fluid imbibing tube between the imbibing position and the rinsing position.

13. The online sampler according to any one of claims 1 to 6, further comprising a volume acquiring module configured to determine a volume of the scanning fluid in the test tube.

14. The online sampler according to claim 13, wherein:

   the volume acquiring module comprises a light source, an image capturing device, and an image analyzer; and

   the light source and the image capturing device are disposed at two sides, respectively, of the test tube;

   the image capturing device is configured to capture an image of the test tube containing the scanning fluid and transmit the image to the image analyzer; and

   the image analyzer is configured to determine the volume of the scanning fluid in the test tube based on the image.

15. The online sampler according to claim 14, wherein the test tube comprises a large-diameter section and a small-diameter section which are connected, and a mouth of the test tube is located at the large-diameter section.

16. The online sampler according to claim 14, further comprising a sampling box having a chamber, wherein the test-tube module and the volume acquiring module are disposed in the chamber.

17. The online sampler according to claim 16, wherein the sampling box is further provided with an air pumping port connected to the chamber.

18. A system for analyzing contaminants, comprising:

a collection system and a mass spectrometer that are integrated, and
the foregoing online sampler according to any one of claims 1 to 17, wherein:

a scanning-fluid nozzle of the collection system is located at a position corresponding to the test-tube module; and
an end of the fluid imbibing tube is configured to imbibe a liquid from the test tube, and another end of the fluid imbibing tube is connected to the mass spectrometer.

19. The system for analyzing contaminants according to claim 18, wherein:

the collection system is configured to collect metallic contaminants through vapor phase decomposition, and/or
the mass spectrometer is an inductively coupled plasma mass spectrometer.

**Figure 1**

**Figure 2**

**Figure 3**

**Figure 4**

**Figure 5**

**Figure 6**

**Figure 7**

**Figure 8**

**Figure 9**

## INTERNATIONAL SEARCH REPORT

| | International application No. |
|---|---|
| | **PCT/CN2021/130879** |

| **A.** | **CLASSIFICATION OF SUBJECT MATTER** |
|---|---|

G01N 1/14(2006.01)i

According to International Patent Classification (IPC) or to both national classification and IPC

| **B.** | **FIELDS SEARCHED** |
|---|---|

Minimum documentation searched (classification system followed by classification symbols)

G01N1/-

Documentation searched other than minimum documentation to the extent that such documents are included in the fields searched

Electronic data base consulted during the international search (name of data base and, where practicable, search terms used)

CNABS, CNTXT, TWTXT, CNKI, WPI, EPODOC, USTXT: 鲁汶仪器, 张怀东, 崔虎山, 程实然, 于翔, 邹志文, 郭德林, 胡冬冬, 许开东, 取样, 沾污, 金属, 升降, 取液, 旋转, 转动, 倒转, 清洁, 试管, 清洗, 烘干, 干燥, 质谱仪, sampl+, metal, impurity, test tube, clean+, mass spectroscopy

| **C.** | **DOCUMENTS CONSIDERED TO BE RELEVANT** |
|---|---|

| Category* | Citation of document, with indication, where appropriate, of the relevant passages | Relevant to claim No. |
|---|---|---|
| X | CN 102269663 A (WUHAN XINXIN SEMICONDUCTOR MANUFACTURING CORP. et al.) 07 December 2011 (2011-12-07) <br> description, paragraphs [0002]-[0071], and figures 1-3 | 1, 13-19 |
| Y | CN 102269663 A (WUHAN XINXIN SEMICONDUCTOR MANUFACTURING CORP. et al.) 07 December 2011 (2011-12-07) <br> description, paragraphs [0002]-[0071], and figures 1-3 | 2-12 |
| Y | CN 108031690 A (YANG XUEFENG) 15 May 2018 (2018-05-15) <br> description, paragraphs [0019]-[0022] | 2-7 |
| Y | CN 102520200 A (BEIJING ZONCI WEIYE TECHNOLOGY DEVELOPMENT CO., LTD.) 27 June 2012 (2012-06-27) <br> description, paragraphs [0053]-[0065] | 8-12 |
| A | CN 102043061 A (AOI SEIKI CO., LTD.) 04 May 2011 (2011-05-04) <br> entire document | 1-19 |
| A | CN 109261672 A (SANMEN CHUNTUO ENVIRONMENTAL PROTECTION TECHNOLOGY CO., LTD.) 25 January 2019 (2019-01-25) <br> entire document | 1-19 |

☑ Further documents are listed in the continuation of Box C.     ☑ See patent family annex.

| | |
|---|---|
| \*   Special categories of cited documents: <br> "A"   document defining the general state of the art which is not considered to be of particular relevance <br> "E"   earlier application or patent but published on or after the international filing date <br> "L"   document which may throw doubts on priority claim(s) or which is cited to establish the publication date of another citation or other special reason (as specified) <br> "O"   document referring to an oral disclosure, use, exhibition or other means <br> "P"   document published prior to the international filing date but later than the priority date claimed | "T"   later document published after the international filing date or priority date and not in conflict with the application but cited to understand the principle or theory underlying the invention <br> "X"   document of particular relevance; the claimed invention cannot be considered novel or cannot be considered to involve an inventive step when the document is taken alone <br> "Y"   document of particular relevance; the claimed invention cannot be considered to involve an inventive step when the document is combined with one or more other such documents, such combination being obvious to a person skilled in the art <br> "&"   document member of the same patent family |

| Date of the actual completion of the international search | Date of mailing of the international search report |
|---|---|
| **21 March 2022** | **13 April 2022** |

| Name and mailing address of the ISA/CN | Authorized officer |
|---|---|
| **China National Intellectual Property Administration (ISA/CN)** <br> **No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

**INTERNATIONAL SEARCH REPORT**

| International application No. |
| --- |
| **PCT/CN2021/130879** |

**C.    DOCUMENTS CONSIDERED TO BE RELEVANT**

| Category* | Citation of document, with indication, where appropriate, of the relevant passages | Relevant to claim No. |
| --- | --- | --- |
| A | CN 210242327 U (LUOHE MEDICAL COLLEGE) 03 April 2020 (2020-04-03)<br>    entire document | 1-19 |
| A | CN 109904089 A (TIANJIN ZHONGHUAN ADVANCED & MATERIAL TECHNOLOGY<br>CO., LTD.) 18 June 2019 (2019-06-18)<br>    entire document | 1-19 |
| A | CN 108655069 A (WUHU ZHONGMENG ELECTRONIC TECHNOLOGY CO., LTD.) 16<br>October 2018 (2018-10-16)<br>    entire document | 1-19 |
| A | CN 106990158 A (LEUVEN INSTRUMENTS NV (BELGIUM)) 28 July 2017 (2017-07-28)<br>    entire document | 1-19 |
| A | CN 102486446 A (SHANGHAI ANCAI INTELLIGENT TECHNOLOGY CO., LTD.) 06 June<br>2012 (2012-06-06)<br>    entire document | 1-19 |
| A | CN 108333381 A (RAYTOR INSTRUMENTS CO., LTD.) 27 July 2018 (2018-07-27)<br>    entire document | 1-19 |
| A | US 2003216238 A1 (HITACHI KOKI CO., LTD.) 20 November 2003 (2003-11-20)<br>    entire document | 1-19 |

Form PCT/ISA/210 (second sheet) (January 2015)

**INTERNATIONAL SEARCH REPORT**
Information on patent family members

International application No.

**PCT/CN2021/130879**

| Patent document cited in search report | | | Publication date (day/month/year) | Patent family member(s) | | | Publication date (day/month/year) |
|---|---|---|---|---|---|---|---|
| CN | 102269663 | A | 07 December 2011 | CN | 102269663 | B | 30 January 2013 |
| CN | 108031690 | A | 15 May 2018 | CN | 110479715 | A | 22 November 2019 |
| | | | | CN | 110479715 | B | 29 June 2021 |
| CN | 102520200 | A | 27 June 2012 | CN | 102520200 | B | 18 September 2013 |
| CN | 102043061 | A | 04 May 2011 | CA | 2717964 | A1 | 20 April 2011 |
| | | | | KR | 20110043483 | A | 27 April 2011 |
| | | | | KR | 101197788 | B1 | 05 November 2012 |
| | | | | JP | 2011107120 | A | 02 June 2011 |
| | | | | TW | 201122454 | A | 01 July 2011 |
| | | | | EP | 2315035 | A2 | 27 April 2011 |
| | | | | US | 2011091988 | A1 | 21 April 2011 |
| CN | 109261672 | A | 25 January 2019 | None | | | |
| CN | 210242327 | U | 03 April 2020 | None | | | |
| CN | 109904089 | A | 18 June 2019 | None | | | |
| CN | 108655069 | A | 16 October 2018 | CN | 108655069 | B | 09 July 2021 |
| CN | 106990158 | A | 28 July 2017 | CN | 106990158 | B | 07 February 2020 |
| CN | 102486446 | A | 06 June 2012 | None | | | |
| CN | 108333381 | A | 27 July 2018 | CN | 207851094 | U | 11 September 2018 |
| US | 2003216238 | A1 | 20 November 2003 | GB | 0305029 | D0 | 09 April 2003 |
| | | | | GB | 2388563 | A | 19 November 2003 |
| | | | | GB | 2388563 | B | 19 May 2004 |
| | | | | US | 6857997 | B2 | 22 February 2005 |
| | | | | DE | 10311329 | A1 | 04 December 2003 |
| | | | | DE | 10311329 | B4 | 23 April 2009 |
| | | | | CN | 1459336 | A | 03 December 2003 |
| | | | | CN | 1291797 | C | 27 December 2006 |
| | | | | JP | 2003334057 | A | 25 November 2003 |
| | | | | JP | 2003334468 | A | 25 November 2003 |
| | | | | JP | 2003337088 | A | 28 November 2003 |
| | | | | JP | 3859137 | B2 | 20 December 2006 |
| | | | | JP | 4110455 | B2 | 02 July 2008 |
| | | | | JP | 4224759 | B2 | 18 February 2009 |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 202110809061